(19) Europäisches Patentamt
European Patent Office
Office européen des brevets

(11) **EP 4 382 099 A1**

(12) **EUROPEAN PATENT APPLICATION**

(43) Date of publication:
**12.06.2024 Bulletin 2024/24**

(21) Application number: **22211365.6**

(22) Date of filing: **05.12.2022**

(51) International Patent Classification (IPC):
*A61K 31/155* (2006.01)   *A61K 9/50* (2006.01)
*A61K 9/48* (2006.01)   *A61K 9/28* (2006.01)
*A61K 9/20* (2006.01)

(52) Cooperative Patent Classification (CPC):
**A61K 31/155; A61K 9/2027; A61K 9/205;
A61K 9/284; A61K 9/2846; A61K 9/2886;
A61K 9/5026; A61K 9/5073; A61K 9/5084**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **ADD Advanced Drug Delivery
Technologies, Ltd.
4133 Pratteln (CH)**

(72) Inventors:
• **Nowak, Mirko
79540 Lörrach (DE)**
• **Grave, Annette
79541 Lörrach (DE)**

(74) Representative: **Breuer, Markus et al
Sendlinger Straße 29
80331 München (DE)**

Remarks:
Claims 16 - 20 are deemed to be abandoned due to
non-payment of the claims fees (Rule 45(3) EPC).

(54) **DRUG LOADED MODIFIED RELEASE PELLETS**

(57) A delayed release formulation for delivery of an active pharmaceutical ingredient (API) to the colon comprising pellets with a core and a layered coating on the core, the core comprising the API and the layered coating comprising a first layer comprising a first film forming polymer and a second layer comprising a second film forming polymer, wherein the first film forming polymer is a film forming polymer providing for a pH-dependent release above pH 7.0 and the second film forming polymer is a film forming polymer providing for a pH-independent release.

EP 4 382 099 A1

**Description**

Pharmaceutical formulations are provided that enable targeted and modified release of an active ingredient, such as metformin or an acid addition salt of metformin, in particular metformin hydrochloride, from drug-loaded pellets in the colon.

The pellets are characterized not only by the targeted release, but also by a high active ingredient load.

Preparation methods are also provided.

**Background**

Oral administration is the most popular form of administering active pharmaceutical ingredients. Numerous formulations have been suggested to formulate drugs for oral administration. These formulations have in common that they release the drug in the intestinal tract after ingestion of the formulation.

For some active pharmaceutical ingredients oral dosage forms are of interest which deliver the ingredient in the colon.

Targeted drug delivery into the colon is desirable for local treatment of a variety of colonic pathologies such as ulcerative colitis, Crohn's disease, amebiasis, colonic cancer, but also for the systemic delivery of drugs.

To reach the colon the pharmaceutical formulation has to transit the oral cavity, the stomach and the small intestine until it reaches the target site, the colon which belongs to the large intestine.

The colon specific drug delivery system should be capable of protecting the drug on its way to the colon. Drug release should occur neither in the stomach nor in the small intestine. Upon release in the colon, the drug is mainly absorbed via the intestinal mucosa.

In order to achieve delivery in the colon, various formulations have been suggested.

NZ 748404 A described a formulation for delivery of a drug to the colon comprising a particle with a core and a coating for the core, the core comprising a drug and the coating comprising a single layer of a mixture of a first material which is susceptible to attack by colonic bacteria and a second material which is a film-forming polymeric material having a pH threshold at about pH 6.5 or above.

US 2021/228487 A1 describes a pharmaceutical formulation for the treatment of inflammatory diseases of the colon. The formulation is a capsule containing pellets, which have multiple coatings, namely an active substance layer directly applied to a starter pellet; a swelling layer directly applied to the active substance layer; a retard layer directly applied to the swelling layer; and an outermost coating which does not dissolve at a pH value <5.5, but dissolves well at a pH value greater than 6.0 and which is directly applied to the retard layer.

CA 3 122 025 A1 describes a formulation for delivery of a drug to the colon which comprises a core comprising a drug and coating for the core. The coating comprises an outer layer and an inner layer. The outer layer comprises a film-forming enteric polymer having a pH threshold at about pH 6 or above and the inner layer comprises a film-forming non-ionic polymer that is soluble in intestinal or gastrointestinal fluid, and a buffer agent.

CN 112791060 A describes a method of producing pharmaceutical compositions for oral administration and colon delivery especially of mesalazine or 5-aminosalicylic acid. The method involves an active ingredient powder application technology and the application of a coating containing at least an anionic (meth)acrylic copolymer soluble at a pH higher than 6 and an anionic (meth)acrylic copolymer soluble at a pH higher than 7.

JO 3574 B1 describes a delayed release formulation comprising a core and a coating for the core. The core comprises a drug and the coating comprises an outer layer which comprises a mixture of a first polymeric material which is susceptible to attack by colonic bacteria, and a second polymeric material which has a pH threshold at about pH 5 or above.

Against this background, there remains a need for formulations which allow colon delivery especially of water-soluble APIs. For instance, there has been a recent interest in the colon release of metformin. Metformin is typically employed in the form of its hydrochloride, which is highly soluble in water.

**Summary of the Invention**

The present invention provides a delayed release formulation for delivery of an active pharmaceutical ingredient (API) to the colon comprising

pellets with a core and a layered coating on the core, the core comprising the API and
the layered coating comprising
a first layer comprising a first film forming polymer and
a second layer comprising a second film forming polymer
wherein the first film forming polymer is a film forming polymer providing for a pH-dependent release above pH 7.0 and
the second film forming polymer is a film forming polymer providing for a pH-independent release.

Preferred embodiments of the invention are defined in the dependent claims.

**Detailed Description of the Invention**

The present invention is based on the finding that a particularly effective control of the release of an active ingredient, such as metformin or an acid addition salt of metformin, in particular metformin hydrochloride, in the colon can be achieved by pellet formulations wherein the release from the pellets is controlled by two different release control mechanisms. According to the invention, the pellets are provided with a layered coating comprising a first layer providing for a pH-dependent release and a second layer providing for a pH-independent release.

The first layer providing for a pH-dependent release comprises a first film forming polymer which provides for a pH-dependent release above pH 7.0.

The second layer comprises a second film forming polymer which provides for a pH-independent release.

The order in which the first layer and the second layer are applied to the core can be chosen.

According to the present invention, release in the colon is not solely dependent on pH increase but is in addition ensured by a retarding polymer.

## Active Ingredients

Active pharmaceutical ingredients that can be formulated according to the invention are those for which a release in the colon is desired.

Formulations according to the invention are in particular suitable for APIs which are soluble in water (from 10 to 30 parts of water required per part of API); freely soluble in water (from 1 to 10 parts of water required per part of API); or even very soluble (less than 1 part of water required per part of API).

Solubility is determined at a temperature of 37°C. Parts of solvent or solute are parts by weight.

An API of particular interest is metformin (1,1-dimethylbiguanide). Metformin can be provided in water-soluble form. Preferred examples are acid addition salts of metformin, in particular metformin hydrochloride (solubility of > 300 g/L).

## Pellet Cores

The cores of the pellets comprised in the delayed release formulation according to the invention contain the active pharmaceutical ingredient.

Typically, at least 90 %wt of the pellets have a core with a size including an optional seal coat in the range of 100 - 2000 $\mu$m as determined by sieve analysis . The range is preferably 200 - 1000 $\mu$m, more preferably 300 - 700 $\mu$m and in particular 315-500 $\mu$m.

In a further preferred aspect of the invention, the cores are round as indicated by a small eccentricity.

The eccentricity can be determined by optical means, for instance, using an Eyecon2® device. This device is a direct imaging particle size analyser that can measure size and shape information of particles within a range of 50-5500 $\mu$m. To obtain images, the particles are illuminated from different angles by red, green, and blue LEDs.

The images obtained are analysed by the software EyePASS@ to determine particle size and shape by calculating the best fitting ellipse. The particle size is given as numeric or volumetric distribution (e.g., Dn50 or Dv50) and the shape is presented as an average eccentricity.

The eccentricity $\varepsilon$ is a measure of how much the fitted ellipse deviates from being circular. It is calculated from the length of the major axis (a) and the length of the minor axis (b) of the ellipse:

$$\sqrt{1 - \frac{b^2}{a^2}}$$

The eccentricity of a circle is zero. The eccentricity of an ellipse which is not a circle is greater than zero but less than 1.

The smaller the eccentricity is the rounder the pellets are.

In a preferred aspect of the invention, the cores including an optional seal coating have a mean eccentricity of 0.45 or below, preferably 0.40 or below, in particular 0.35 or below.

In a preferred aspect of the invention, the cores contain a high proportion of API. For instance, the core comprises at least 50 %wt of API, preferably at least 70%wt of API, more preferably at least 90 %wt of API, in particular at least 95 %wt of API, based on the weight of all components of the core .

## Matrix Pellets as Cores

The cores of the pellets comprised in the delayed release formulation according to the invention can be matrix pellets comprising the API and one or more binders.

Binders which can be used for preparing the matrix pellets are usually polymers or polymer compositions.

Suitable polymers include Carbopol 974P NF (molecular weight >1000 g/mol); Chitosan (molecular weight 50000-190000 g/mol) and HPMC (Pharmacoat 603) (molecular weight 10000-150000 g/mol).

Cores can be prepared by a process using a spouted bed apparatus as described in DE 103 22 062 A1. The process involves introducing an aqueous spraying solution containing the polymer and the API into a solids flow of a spouted bed apparatus, as well as an associated apparatus. For this approach, it is characteristic that at least one circular solids flow is formed in the axial direction of the reaction space of the spouted bed apparatus, for which the inlet air required for forming the solids flow is fed through a gap in the lower region and in the axial direction of the reaction space and the fluid is introduced by means of one or more single or multiple-component nozzles at one or more positions into the solids flow. Therefore, the flow conditions in the injection region can be set, so that the fluid can be introduced selectively and adjustably into the solids flow. The resulting end product distinguishes itself through approximately equal grain size with equal material properties.

In this way, matrix pellets can be prepared which have a round shape as characterized by a low eccentricity and which contain only 3-5 wt% of binder.

**Drug Layered Inert Starters as Cores**

The cores of the pellets comprised in the delayed release formulation according to the invention can be drug layered inert starters, i.e., starter pellets free of active ingredient provided with a layer comprising a drug and one or more binders.

Any pellet suitable for pharmaceutical use which does not contain an API can be employed as an inert starter.

Suitable inert starters are, for instance, glass beads and pellets made from sucrose, mannose or microcrystalline cellulose. Inert starters made of microcrystalline cellulose, are preferred. Such pellets are commercially available under the name Cellets®.

The size of the cores is not limited. Suitable sizes are in the range from 10 μm to 2000 μm, for example in the range from 50 μm to 1500 μm and preferably 100 μm to 1000 μm, the size may be determined by sieve analysis. In particular, pellets from a sieve fraction of 500-710 μm may be used.

The inert starters, for instance, inert starter pellets based on microcrystalline cellulose (Cellets®) are provided with an API containing coating. For applying the API containing coating, the API is combined with one or more excipients. These excipients typically comprise a film forming polymer. Suitable film forming polymers are preferably water-soluble.

A particularly preferred film forming polymer is HPMC having a molecular weight of 10000 to 150000, such as Pharmacoat® 603 (Shin Etsu).

The API layering can be carried out by any suitable technique. A preferred technique is the Wurster coating technique. In this approach, an aqueous liquid containing the API and the film-forming polymer and optionally an anti-tacking agent such as colloidal silicone dioxide or talc is sprayed into a Wurster coated containing a fluidized bed containing the starter pellets.

**Seal Coats**

To avoid any incompatibility of active ingredients and other components of the core on the one hand and the coating polymers on the other hand the cores can be seal coated. The seal coat is typically water soluble and in particular based on a water-soluble film forming polymer.

The seal coat preferably does not retard or otherwise control the release of the API.

Preferably, the cores which are optionally provided with a seal coating, show immediate release properties. In other words, the cores, optionally comprising the seal coat, but before application of the layered coating, release more than 90 %wt of the API after 15 min when subjected to a dissolution test in a buffer pH 6.8 for 45 minutes by using paddle apparatus with 75 rpm.

Water-soluble film forming polymers which can be used for seal coating are, for example, HPMC, chitosan, or Carbopol.

A particularly preferred seal coat is based on HPMC having a molecular weight of 10000 to 150000, such as Pharmacoat® 603 (Shin Etsu).

**Release Control Coatings**

The pellets comprised in the delayed release formulation according to the invention comprise a core which is provided with a layered coating. This layered coating comprises a first layer comprising a first film forming polymer and a second layer comprising a second film forming polymer. The first film forming polymer is a film forming polymer providing for a pH-dependent release above pH 7.0. The second film forming polymer is a film forming polymer providing for a pH-independent release.

The first film forming polymer can be a polymer comprising anionic repeating units based on (meth)acrylic acid. For instance, it can be a copolymer of methyl acrylate, methyl methacrylate and methacrylic acid. An example of such a copolymer is poly(methyl acrylate-co-methyl methacrylate-co-methacrylic acid) 7:3:1.

An example of the molecular weight is 280000.

The first film forming polymer can be applied in the form of an aqueous dispersion.

A suitable film-forming dispersion is commercially available as Eudragit® FS30 D (Evonik).

The second film forming polymer is insoluble in water or has a low water solubility.

The second film forming polymer can be a polyvinylacetate, in particular polyvinylacetate having a molecular weight of 200000.

It can be used in the form of Poly (Vinyl Acetate) Dispersion 30 per Cent Ph. Eur. or Polyvinyl Acetate Dispersion USP.

A suitable composition is commercially available as Kollicoat® SR30 D (BASF). The dispersion contains about 27% polyvinyl acetate, 2.7% povidone and 0.3% sodium lauryl sulfate.

The release control achieved depends on coating level achieved.

According to the invention, the coating level is preferably adjusted based on the surface area of the cores (including any optional seal coating).

Generally, the pH-dependent coating is applied at a level of 2-5 mg / cm$^2$ and the pH-independent coating is applied at a level of 0.5- 2.5 mg / cm$^2$. As used herein, coating level refers to the total amount of solids applied by the coating process. This amount can be determined as the coating weight gain.

In one embodiment, a pellet as contained in the formulation according to the invention comprises, from inside to outside, the core, the first layer (which provides for a pH-dependent release) and the second layer (which provides for a pH-independent sustained release).

In this embodiment, the first film forming polymer is preferably applied at a coating level in the range of 2 to 3 mg/cm$^2$ and the second film forming polymer is preferably applied at a coating level in the range of 0.5 to 2.5 mg/cm$^2$.

In another embodiment, a pellet as contained in the formulation according to the invention comprises, from inside to outside, the core, the second layer (which provides for a pH-independent sustained release) and the first layer (which provides for a pH-dependent release).

In this embodiment, the first film forming polymer is preferably applied at a coating level in the range of 2 to 5 mg/cm$^2$ and the second film forming polymer is preferably applied at a coating level in the range of 0.5 to 2.5 mg/cm$^2$.

**Release Profiles**

The API should not be released within the first 180 minutes respectively until the pH reaches 7.2. Between 180 - 300 minutes the API should be released slowly but completely.

A formulation according to the present invention meets at least 3 of the following criteria for the release of the API: release of not more than 10 wt% after 120 min; release of not more than 10 wt% after 180 min; release of between 10 and 30 wt% after 210 min; release of between 40 and 60 wt% after 225 min; release of between 60 and 80 wt% after 240 min; more than 80 wt% after 270 min; wherein release is determined under the following conditions: 0.1 M potassium dihydrogen phosphate (2 hours), followed in buffer pH 5.5 (1 hour) and buffer pH 7.2 (2 hours).

**Examples**

Example 1.) and 2.)

Pellets layered to an API content of 50 % using Pharmacoat 603 as binder, applying Wurster technology; first coating Eudragit FS 30 D (coating level 30 %), second coating Kollicoat SR 30 D (coating level 10 % or 20 %)

Example 3.) and 4.)

Pellets layered to an API content of 50 % using Pharmacoat 603 as binder, applying Wurster technology; first coating Kollicoat SR 30 D (coating level 10 %), second coating Eudragit FS 30 D (coating level 30 % or 50 %)

Example 5.), 6.) and 7.)

Pellets layered to an API content of 50 % using Pharmacoat 603 as binder, applying Wurster technology; seal coating with Carbopol 974P NF (coating level 5 %); first coating Eudragit FS 30 D (coating level 30 %), second coating Kollicoat SR 30 D (coating level 10%, 15% or 20 %)

Example 8.), 9.) and 10.)

Matrix Pellets produced by ProCell technology with an API content of 95 % using Carbopol 974P NF as binder; first coating Eudragit FS 30 D (coating level 30 %), second coating Kollicoat SR 30 D (coating level 10 %, 15 % or 20 %)

Example 11.) and 12.)

Matrix Pellets produced by ProCell technology with an API content of 99 % using Chitosan as binder; seal coating with Pharmacoat 603 (coating level 20 %); first coating Eudragit FS 30 D (coating level 30 %), second coating Kollicoat SR 30 D (coating level 15 % or 20 %).

**Claims**

1. A delayed release formulation for delivery of an active pharmaceutical ingredient (API) to the colon comprising

   pellets with a core and a layered coating on the core,
   the core comprising the API and
   the layered coating comprising
   a first layer comprising a first film forming polymer and
   a second layer comprising a second film forming polymer
   wherein the first film forming polymer is a film forming polymer providing for a pH-dependent release above pH 7.0 and
   the second film forming polymer is a film forming polymer providing for a pH-independent release.

2. The formulation according to claim 1, wherein the API is soluble in water so that not more than from 10 to 30 parts of water is required per part of API to form a solution, wherein all parts are parts by weight.

3. The formulation according to claim 2, wherein the API is a water-soluble form of metformin.

4. The formulation according to claim 2, wherein the API is metformin hydrochloride.

5. The formulation according to any of claims 1 to 4, wherein at least 90 %wt of the pellets have a core with a size including an optional seal coat in the range of 100 - 2000 μm as determined by sieve analysis.

6. The formulation according to any of claims 1 to 5, wherein the core comprises an inert nucleus and, on said nucleus, a layer containing the API.

7. The formulation according to any of claims 1 to 5, wherein the core comprises a matrix pellet.

8. The formulation according to any of claims 1 to 7, wherein the core comprises at least 50 %wt of API, preferably at least 70%wt of API, more preferably at least 90 %wt of API, based on the weight of all components of the core.

9. The formulation according to any of claims 1 to 8, wherein the cores including optional seal coating have a mean eccentricity of 0.45 or below, preferably 0.40 or below, in particular 0.35 or below.

10. The formulation according to any of claims 1 to 9, wherein the cores, optionally comprising the seal coat, but before application of the layered coating, release more than 90 %wt of the API after 15 min when subjected to a dissolution test in a buffer pH 6.8 for 45 minutes by using paddle apparatus with 75 rpm.

11. The formulation according to any of claims 1 to 10, wherein the first film forming polymer the first film forming polymer comprises anionic repeating units based on (meth)acrylic acid.

12. The formulation according to claim 11, wherein the first film forming polymer is a copolymer of methyl acrylate, methyl methacrylate and methacrylic acid.

13. The formulation according to claim 12, wherein the first film forming polymer is poly(methyl acrylate-co-methyl methacrylate-co-methacrylic acid) 7:3:1.

14. The formulation according to any of claims 1 to 13, wherein the second film forming polymer is polyvinyl acetate.

15. The formulation according to any of claims 1 to 13, wherein the pH-dependent coating is applied at a level of 2-5 mg / cm$^2$ and the pH-independent coating is applied at a level of 0.5-2.5 mg / cm$^2$.

16. The formulation according to any of claims 1 to 14, wherein the pellet comprises, from inside to outside, the core, the first layer (which provides for a pH-dependent release) and the second layer (which provides for a pH-independent sustained release).

17. The formulation according to claim 16, wherein the first film forming polymer is applied at a coating level in the range of 2 to 3 mg/cm$^2$ and the second film forming polymer is applied at a coating level in the range of 0.5 to 2.5 mg/cm$^2$.

18. The formulation according to any of claims 1 to 14, wherein the pellet comprises, from inside to outside, the core, the second layer (which provides for a pH-independent sustained release) and the first layer (which provides for a pH-dependent release).

19. The formulation according to claim 18, wherein the first film forming polymer is applied at a coating level in the range of 2 to 5 mg/cm$^2$ and the second film forming polymer is applied at a coating level in the range of 0.5 to 2.5 mg/cm$^2$.

20. The formulation according to any of claims 1 to 19, wherein the formulation meets at least 3 of the following criteria for the release of the API: release of not more than 10 wt% after 120 min; release of not more than 10 wt% after 180 min; release of between 10 and 30 wt% after 210 min; release of between 40 and 60 wt% after 225 min; release of between 60 and 80 wt% after 240 min; more than 80 wt% after 270 min; wherein release is determined under the following conditions: 0.1 M potassium dihydrogen phosphate (2 hours), followed in buffer pH 5.5 (1 hour) and buffer pH 7.2 (2 hours).

Europäisches
Patentamt

European
Patent Office

Office européen
des brevets

# EUROPEAN SEARCH REPORT

**Application Number**

EP 22 21 1365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/228487 A1 (WILHELM RUDOLF [DE] ET AL) 29 July 2021 (2021-07-29) | 1,2,5-15 | INV.<br>A61K31/155 |
| Y | * paragraphs [0078], [0136]; claim 1; figures 4,5 *<br>* paragraphs [0085], [0089]; claim 2 * | 3,4 | A61K9/50<br>A61K9/48<br>A61K9/28<br>A61K9/20 |
| X | CA 3 122 025 A1 (TILLOTTS PHARMA AG [CH]) 11 June 2020 (2020-06-11) | 1,2, 5-11,15 | |
| Y | * pages 26-37; claim 1; examples 1-3 * | 3,4, 12-14 | |
| X | WO 2022/172225 A1 (LYRUS LIFE SCIENCES PVT LTD [IN]; NOKHA TRADING LLP [IN] ET AL.) 18 August 2022 (2022-08-18) | 1-11,14, 15 | |
| Y | * pages 21-22; claim 1; example 3 *<br>* claim 8 * | 12,13 | |
| Y | WO 2021/115649 A1 (EVONIK OPERATIONS GMBH [DE]) 17 June 2021 (2021-06-17)<br>* page 10, lines 3-7 *<br>* page 10, lines 30-32 *<br>* claim 4 * | 3,4, 12-14 | |

| | | | TECHNICAL FIELDS SEARCHED (IPC) |
|---|---|---|---|
| | | | A61K |

The present search report has been drawn up for all claims

| Place of search | Date of completion of the search | Examiner |
|---|---|---|
| Munich | 18 August 2023 | Cetinkaya, Murat |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 21 1365

This annex lists the patent family members relating to the patent documents cited in the above-mentioned European search report.
The members are as contained in the European Patent Office EDP file on
The European Patent Office is in no way liable for these particulars which are merely given for the purpose of information.

18-08-2023

| Patent document cited in search report | | | Publication date | Patent family member(s) | | Publication date |
|---|---|---|---|---|---|---|
| US 2021228487 | A1 | | 29-07-2021 | AU | 2019326752 A1 | 28-01-2021 |
| | | | | CA | 3108257 A1 | 27-02-2020 |
| | | | | CN | 112512513 A | 16-03-2021 |
| | | | | DK | 3840734 T3 | 26-09-2022 |
| | | | | EA | 202190565 A1 | 01-06-2021 |
| | | | | EP | 3613414 A1 | 26-02-2020 |
| | | | | EP | 3840734 A1 | 30-06-2021 |
| | | | | ES | 2927000 T3 | 31-10-2022 |
| | | | | HR | P20221162 T1 | 25-11-2022 |
| | | | | HU | E060169 T2 | 28-02-2023 |
| | | | | IL | 279825 A | 01-03-2021 |
| | | | | JP | 2022503581 A | 12-01-2022 |
| | | | | LT | 3840734 T | 25-10-2022 |
| | | | | PL | 3840734 T3 | 19-12-2022 |
| | | | | PT | 3840734 T | 19-09-2022 |
| | | | | RS | 63628 B1 | 31-10-2022 |
| | | | | SI | 3840734 T1 | 28-10-2022 |
| | | | | US | 2021228487 A1 | 29-07-2021 |
| | | | | WO | 2020039017 A1 | 27-02-2020 |
| | | | | ZA | 202100512 B | 23-02-2022 |
| CA 3122025 | A1 | | 11-06-2020 | AR | 117682 A1 | 25-08-2021 |
| | | | | AU | 2019394086 A1 | 17-06-2021 |
| | | | | BR | 112021010915 A2 | 24-08-2021 |
| | | | | CA | 3122025 A1 | 11-06-2020 |
| | | | | CL | 2021001445 A1 | 21-01-2022 |
| | | | | CN | 113164401 A | 23-07-2021 |
| | | | | CO | 2021008688 A2 | 30-07-2021 |
| | | | | CR | 20210365 A | 03-11-2021 |
| | | | | EA | 202191574 A1 | 13-09-2021 |
| | | | | EP | 3662902 A1 | 10-06-2020 |
| | | | | EP | 3890709 A1 | 13-10-2021 |
| | | | | GE | P20237517 B | 26-06-2023 |
| | | | | IL | 283721 A | 29-07-2021 |
| | | | | JP | 2022511118 A | 28-01-2022 |
| | | | | KR | 20210100664 A | 17-08-2021 |
| | | | | MA | 54373 A | 16-03-2022 |
| | | | | SG | 11202105695W A | 29-06-2021 |
| | | | | TW | 202038915 A | 01-11-2020 |
| | | | | US | 2022016038 A1 | 20-01-2022 |
| | | | | WO | 2020115254 A1 | 11-06-2020 |
| WO 2022172225 | A1 | | 18-08-2022 | CA | 3206193 A1 | 18-08-2022 |
| | | | | WO | 2022172225 A1 | 18-08-2022 |
| WO 2021115649 | A1 | | 17-06-2021 | BR | 112022011295 A2 | 06-09-2022 |

For more details about this annex : see Official Journal of the European Patent Office, No. 12/82

EPO FORM P0459

page 1 of 2

## ANNEX TO THE EUROPEAN SEARCH REPORT
### ON EUROPEAN PATENT APPLICATION NO.

**EP 22 21 1365**

This annex lists the patent family members relating to the patent documents cited in  the above-mentioned European search report.
The members are as contained in the European Patent Office EDP file on
The European Patent Office is in no way liable for these particulars which are merely given for the purpose of information.

**18-08-2023**

| Patent document cited in search report | Publication date | Patent family member(s) | | Publication date |
|---|---|---|---|---|
| | | CA | 3160862 A1 | 17-06-2021 |
| | | CN | 114828832 A | 29-07-2022 |
| | | EP | 4072531 A1 | 19-10-2022 |
| | | IL | 293653 A | 01-08-2022 |
| | | JP | 2023505883 A | 13-02-2023 |
| | | KR | 20220113941 A | 17-08-2022 |
| | | US | 2023048354 A1 | 16-02-2023 |
| | | WO | 2021115649 A1 | 17-06-2021 |

EPO FORM P0459

For more details about this annex : see Official Journal of the European Patent Office, No. 12/82

**page 2 of 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- NZ 748404 A
- US 2021228487 A1
- CA 3122025 A1
- CN 112791060 A
- DE 10322062 A1